(19) **Europäisches Patentamt / European Patent Office / Office européen des brevets**

(11) **EP 4 180 074 B1**

(12) **FASCICULE DE BREVET EUROPEEN**

(45) Date de publication et mention de la délivrance du brevet:
**23.07.2025 Bulletin 2025/30**

(21) Numéro de dépôt: **22197169.0**

(22) Date de dépôt: **22.09.2022**

(51) Classification Internationale des Brevets (IPC):
**A61M 16/00** (2006.01)    *A61M 39/10* (2006.01)

(52) Classification Coopérative des Brevets (CPC):
**A61M 16/0051; A61M 16/024; A61M 39/10;**
A61M 2016/0039; A61M 2039/1005; A61M 2205/15

(54) **DÉTECTION DU DÉBRANCHEMENT DU CIRCUIT PATIENT D'UN VENTILATEUR MÉDICAL À PARTIR DE LA COMPLIANCE**

ERKENNUNG DER TRENNUNG DES PATIENTENKREISLAUFS EINES MEDIZINISCHEN BEATMUNGSGERÄTES AUS DER NACHGIEBIGKEIT

DETECTING MEDICAL VENTILATOR PATIENT CIRCUIT DISCONNECT FROM COMPLIANCE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.11.2021 FR 2112042**

(43) Date de publication de la demande:
**17.05.2023 Bulletin 2023/20**

(73) Titulaire: **Air Liquide Medical Systems**
**92182 Antony Cedex (FR)**

(72) Inventeurs:
• **POMIER, Romain**
**92182 Antony Cedex (FR)**
• **DERMEL, Marius**
**92182 Antony Cedex (FR)**
• **JACQUOT, Eric**
**92182 Antony Cedex (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
**WO-A1-2016/103122    WO-A1-82/01654**
**US-A- 4 351 344**

**Description**

L'invention concerne une détection du débranchement du circuit patient d'un ventilateur médical alimentant un patient en gaz respiratoire basée sur une estimation de la compliance.

Un ventilateur médical est un appareil d'assistance respiratoire servant à délivrer une aide respiratoire, c'est-à-dire une ventilation artificielle, à un patient souffrant de troubles ou insuffisances respiratoires plus ou moins sévères, pouvant résulter de différentes pathologies ou analogues. Certains patients ayant des pathologies sévères peuvent rester ventilés en milieu hospitalier, pendant plusieurs jours, même plusieurs mois.

Pendant son fonctionnement, le ventilateur médical délivre un gaz respiratoire au patient, par exemple de l'air ou de l'air enrichi en oxygène, via un circuit patient comprenant un ou des conduits servant à véhiculer le gaz, et par ailleurs opère un suivi, i.e. monitorage, de paramètres ventilatoires comme par exemple la pression du gaz, la pression œsophagienne du patient, les volumes de gaz échangés, le débit de gaz... et peut déclencher une alarme sonore ou visuelle pour alerter le personnel soignant en cas de dysfonctionnement du ventilateur ou de problème lié à la ventilation du patient, en particulier en cas de débranchement accidentel du circuit patient.

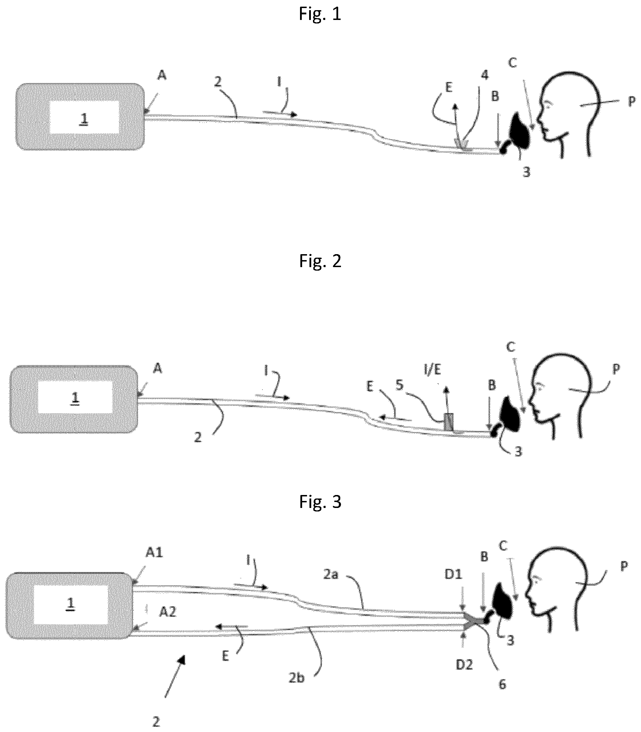

En effet, pendant l'utilisation du ventilateur, il peut survenir une déconnexion ou débranchement accidentel du circuit patient, par exemple au niveau de la liaison entre le ventilateur et le circuit patient, entre le circuit patient et l'interface respiratoire patient alimentant le patient en gaz respiratoire, typiquement un masque respiratoire nasal, buccal ou facial (i.e. bucco-nasal) ou une sonde d'intubation trachéale, ou encore en les branches inspiratoire et/ou expiratoire et la pièce de jonction en Y d'un circuit patient à double branche, comme illustré sur les Fig. 1 à Fig. 3 et détaillé ci-après.

En général, le ventilateur comprend des moyens de détection de débranchement de circuit et des moyens d'alarme servant à détecter tout débranchement du circuit patient et ensuite à prévenir le personnel soignant ou le patient par déclenchement d'une alarme dédiée afin de leur permettre de prendre les mesures adéquates, à savoir une reconnexion des éléments déconnectés.

Or, en pratique, on assiste parfois à des fausses alarmes, c'est-à-dire que le ventilateur détecte par erreur un débranchement alors que tous les éléments sont correctement raccordés.

Il existe plusieurs méthodes de détection d'un débranchement du patient.

Toutefois, ces méthodes ne sont pas idéales car elles nécessitent une prise en compte du type exact de circuit patient utilisé et de ses caractéristiques propres. En effet, un circuit patient comprend des caractéristiques particulières qui sont fonction du patient à traiter, c'est-à-dire qui diffèrent selon que le patient est un adulte, un enfant, un nourrisson...

Or, si l'IHM ou interface homme-machine (e.g. boutons, touches, écran....) d'un ventilateur permet souvent à l'utilisateur d'indiquer la catégorie de patient à traiter (i.e. adulte, enfant ou nourrisson), elle ne permet pas de prendre en compte la référence exacte du circuit patient utilisé, donc de ses particularités, ce qui nuit à une bonne détection des débranchements faute de disposer de toutes les informations nécessaires.

On connait par ailleurs WO-A-82/01654 qui décrit un ventilateur comprenant un capteur de pression servant à déterminer la pression dans le but de suivre la compliance pulmonaire du patient afin de détecter tout risque de pneumothorax ou analogue. La compliance pulmonaire est déterminée uniquement à partir de la pression mesurée par un capteur de pression. Toutefois, la valeur de compliance ne sert pas à déterminer une éventuelle déconnexion d'un élément du circuit du ventilateur.

Le problème est dès lors de proposer une méthode de détection améliorée permettant de détecter efficacement les déconnexions réelles d'un (ou des) élément du circuit tout en minimisant les fausses détections de manière à réduire ou éliminer le nombre de déclenchements de fausses alarmes.

Détecter plus efficacement les débranchements d'un ou plusieurs éléments du circuit patient est aussi un enjeu pour le confort du patient. En effet, en cas d'une déconnexion d'un tel débranchement, le ventilateur doit pouvoir le détecter rapidement et passer immédiatement dans un état ou mode spécifique dit « de débranchement » ou « de déconnexion », impliquant par exemple une diminution de la vitesse de la turbine délivrant le gaz, pour éviter ou minimiser les risques de projection de sécrétions, une consommation électrique et de gaz ($O_2$ par exemple) inutile..., pour réduire le bruit acoustique, ... et autres, et ce, dans le but de ne pas nuire au confort du patient.

La solution de l'invention porte alors sur un ventilateur médical comprenant :

- un circuit de gaz interne pour acheminer du gaz comprenant une sortie de gaz,
- au moins un capteur de débit et au moins un capteur de pression agencés sur le circuit de gaz interne pour opérer des mesures de débit et de pression au sein dudit circuit de gaz interne,
- des moyens de pilotage à au moins un microprocesseur configurés pour traiter les mesures de débit et de pression opérées par lesdits capteurs de débit et de pression,
- un circuit patient raccordé fluidiquement à la sortie de gaz du circuit de gaz interne, et
- une interface respiratoire raccordée fluidiquement au circuit patient,

caractérisé en ce que les moyens de pilotage sont configurés pour :

a) déterminer une valeur de compliance C(t) à partir d'au moins des mesures de débit et de pression opérées par lesdits capteurs de débit et de pression,

b) comparer la valeur de compliance C(t) déterminée à une valeur de compliance maximale ($C_{max}$) donnée,

c) déterminer un débranchement du circuit patient ou de l'interface respiratoire lorsque la valeur de compliance C(t) est supérieure ou égale à la valeur de compliance maximale ($C_{max}$) donnée (i.e. $C(t) \geq C_{max}$), et

d) déclencher une alarme lorsqu'un débranchement du circuit patient ou de l'interface respiratoire (i.e. l'un, l'autre ou des deux) est déterminé.

D'une façon générale, la compliance pulmonaire reflète l'élasticité ou distensibilité des poumons, c'est-à-dire leur capacité à absorber un volume supplémentaire de gaz selon une variation de pression ou encore, selon une définition courante, le rapport d'une variation du volume pulmonaire à la variation de la pression de gaz (e.g. air) correspondante. Si les poumons se distendent facilement à l'inspiration et reprennent aisément leur volume initial à l'expiration du patient, alors la compliance est considérée comme bonne ou normale. Une compliance « anormale », e.g. trop élevée ou trop faible, est le reflet de maladies ou troubles respiratoires.

Selon le mode de réalisation considéré, le ventilateur médical de l'invention peut comprendre l'une ou plusieurs des caractéristiques suivantes :

- il comprend des moyens de fixation ou de sélection de compliance maximale pour fixer ou sélectionner une valeur de compliance maximale ($C_{max}$), de préférence comprise entre 20 et 600 mL/cmH2O, de préférence au moins 40 mL/cmH2O, typiquement au moins 100 mL/cmH2O, par exemple 100, 200 ou 300 mL/cmH2O.
- il comprend des moyens de sélection de catégorie de patient permettant de sélectionner une catégorie de patient choisie parmi adulte, enfant et nourrisson, ladite sélection de catégorie de patient conduisant à une sélection automatique d'une valeur de compliance maximale ($C_{max}$), de préférence comprise entre 20 et 600 mL/cmH2O.
- les moyens de pilotage sont configurés pour calculer un volume de gaz *(Vti)* délivré au patient pendant la durée d'une phase inspiratoire, aussi appelé cycle insufflatoire ou cycle ventilatoire, durant laquelle le ventilateur fournit le gaz au patient et le patient inhale le gaz.
- les moyens de pilotage sont configurés pour déterminer le volume de gaz *(Vti)* à partir des mesures de débit (Q) opérées le cycle ventilatoire, i.e. durant la phase inspiratoire.
- les moyens de pilotage sont configurés pour déterminer le volume de gaz *(Vti)* par intégration (d'au moins une partie) des mesures de débit (Q) pendant (d'au moins une partie) la durée d'un cycle insufflatoire, c'est-à-dire
- les moyens de pilotage sont configurés pour calculer la valeur de compliance C(t) à partir de la formule suivante :

$$C(t) = \frac{Vti}{P_{prox}\big(Fin_{inspi}\big) - P_{prox}\big(Deb_{inspi}\big)}$$

Où :

- $P_{prox}$(*Fin_{inspi}*) est la pression proximale en fin d'inspiration,
- $P_{prox}$(*Deb_{inspi}*) est la pression proximale en début d'inspiration, et
- V*ti* est le volume de gaz délivré (par le ventilateur) au patient sur 1 cycle ventilatoire, c'est-à-dire pendant la durée de la phase inspiratoire pendant laquelle du gaz est délivré au patient.

- la durée de la phase inspiratoire ou cycle ventilatoire est typiquement de quelques secondes, typiquement moins de 6 secondes, par exemple de 1 à 5 secondes environ, typiquement entre 1 et 4 secondes environ, par exemple de 1 à 2 secondes environ.
- les moyens de sélection de catégorie de patient comprennent un organe de sélection manuelle et/ou un écran d'affichage, typiquement une IHM.
- le circuit patient comprend un ou deux conduits de gaz agencés en parallèle, c'est-à-dire un circuit patient à une ou deux branches.
- le circuit patient est relié directement à interface respiratoire ou indirectement via une pièce de jonction ou de raccordement, typiquement une pièce en Y.
- le circuit patient comprend un ou plusieurs conduits souples, i.e. un ou des tuyaux flexibles selon qu'il est à simple branche ou double branche.
- le circuit patient est un circuit à double branche comprenant deux conduits souples agencés en parallèle venant se raccorder à une pièce de jonction, telle une pièce en Y, située entre les deux conduits souples et l'interface patient.
- le circuit patient est un circuit à double branche comprenant une branche inspiratoire et une branche expiratoire.

- la source de gaz est une micro-soufflante motorisée, i.e. comprenant un moteur électrique, aussi appelée soufflante, turbine ou compresseur.
- la micro-soufflante motorisée est configurée pour délivrer un gaz respiratoire de type air ou air enrichi en oxygène (i.e. mélange air/$O_2$).
- les moyens de pilotage sont configurés pour piloter la micro-soufflante motorisée, en particulier les phases d'accélérations et de freinage/décélération dudit moteur.
- les moyens de pilotage sont configurés pour piloter la micro-soufflante pour délivrer du gaz au patient pendant la durée du cycle ventilatoire.
- le circuit de gaz interne comprend au moins un conduit ou passage de gaz.
- lorsque le circuit patient est à simple branche, les moyens de pilotage sont configurés pour déterminer un débranchement du circuit patient ou de l'interface respiratoire situé au niveau de la connexion entre le ventilateur et le circuit patient, et/ou au niveau de la connexion entre le circuit patient et l'interface respiratoire, et/ou au niveau de la région de contact entre l'interface respiratoire et le visage du patient.
- lorsque le circuit patient est à double branche (i.e. une branche inspiratoire et une branche expiratoire), les moyens de pilotage sont configurés pour déterminer un débranchement du circuit patient ou de l'interface respiratoire situé au niveau de la connexion entre le ventilateur et l'une ou l'autre des extrémités amont des deux branches (i.e. branche inspiratoire et branche expiratoire) du circuit patient, et/ou de l'une ou l'autre des connexions entre l'une ou l'autre des extrémités aval des deux branches du circuit et une pièce de jonction, typiquement une pièce en Y, et/ou au niveau de la connexion entre la pièce de jonction (e.g. pièce en Y) et l'interface respiratoire, et/ou au niveau de la région de contact entre l'interface respiratoire et le visage du patient.
- les moyens de pilotage comprennent un ou plusieurs (micro)processeurs, typiquement un microcontrôleur.
- les moyens de pilotage comprennent un (ou plusieurs) microprocesseur(s) agencé(s) sur au moins une carte électronique.
- les moyens de pilotage comprennent un (ou plusieurs) microprocesseur(s) mettant en œuvre au moins un algorithme.
- il comprend en outre des moyens d'alimentation électrique alimentant en courant électrique le ou les composants nécessitant de l'électricité pour fonctionner, notamment les moyens de pilotage.
- les moyens d'alimentation électrique comprennent des moyens de raccordement au secteur (110/220V), tels que câble(s) électrique(s) et/ou prise secteur.
- il comprend en outre une carcasse ou coque externe rigide, par exemple en polymère ou en métal.
- il comprend en outre des moyens de mémorisation, par exemple une mémoire informatique ou analogue, pour mémoriser, i.e. stocker, des données, des valeurs, des informations ou autres.
- les moyens de mémorisation sont configurés pour mémoriser au moins une valeur compliance maximale ($C_{max}$).
- les moyens de pilotage, typiquement le processeur, sont configurés pour déclencher une alarme sonore et/ou visuelle en cas de débranchement du circuit, c'est-à-dire quand un débranchement est détecté.
- l'alarme visuelle comprend un message d'alarme s'affichant sur l'écran d'affichage, l'allumage d'un dispositif d'alerte lumineux, tels une ou des LEDs par exemple.
- l'alarme sonore comprend l'émission d'un signal sonore audible par l'utilisateur, par exemple diffusé via un haut-parleur du ventilateur ou analogue.

L'invention va maintenant être mieux comprise grâce à la description détaillée suivante, faite à titre illustratif mais non limitatif, en référence aux figures annexées parmi lesquelles :

Fig. 1 schématise les sites de débranchement d'un circuit patient à simple branche à valve expiratoire,

Fig. 2 schématise les sites de débranchement d'un circuit patient à simple branche dit « à fuites »,

Fig. 3 schématise les sites de débranchement d'un circuit patient à double branche, et

Fig. 4 schématise un ventilateur médical selon l'invention.

Fig. 1 et Fig. 2 schématisent différents débranchements possibles d'un circuit patient 2 à simple branche, e.g. un conduit flexible ou analogue, reliant fluidiquement un ventilateur médical 1 à une interface respiratoire 3, tel un masque respiratoire ou une sonde trachéale. Le circuit patient 2 permet d'acheminer le gaz respiratoire, tel de l'air ou un mélange air/O2, délivré par le ventilateur médical 1 jusqu'au patient P alors de l'administrer par inhalation au patient P, pendant ses phases inspiratoires.

Sur Fig. 1, la branche unique du circuit patient 2 comprend une valve expiratoire 4 servant à évacuer vers l'atmosphère, le gaz riche en $CO_2$ expiré par le patient P pendant ses phases expiratoires, alors que sur Fig. 2, la branche unique du circuit patient 2 comprend un orifice de fuite 5 relié à l'atmosphère. Alternativement, l'orifice de fuite 5 peut être aussi situé sur l'interface respiratoire 3, tel un masque. Les flèches I et E donnent le sens de circulation des gaz dans le circuit patient 2, pendant les phases inspiratoires (I) et phases expiratoires (E), y compris au travers de la valve expiratoire 4 et de l'orifice de fuite 5.

Dans ces deux modes de réalisation, les débranchements du circuit patient 2 à simple branche peuvent se produire au niveau de la connexion A entre ventilateur 1 et circuit 2, et/ou au niveau de la connexion B entre circuit 2 et interface respiratoire 3 et/ou au niveau de la région de contact C entre interface respiratoire 3 et visage du patient P.

Fig. 3 est analogue aux Fig. 1 et Fig. 2, à l'exception du fait qu'elle schématise les différents débranchements possibles d'un circuit patient 2 à double branche 2a, 2b, e.g. deux conduits flexibles agencés en parallèle ou analogue, reliant fluidiquement le ventilateur médical 1 l'interface respiratoire 3, tel un masque respiratoire, une sonde trachéale ou autre, par l'intermédiaire d'une pièce de jonction 6 en forme de Y, généralement appelée « pièce en Y ». Le circuit patient 2 comprend une branche inspiratoire 2a acheminant le gaz depuis le ventilateur 1 vers le patient P (sens de la flèche I) et une branche expiratoire 2a acheminant le gaz riche en $CO_2$ expiré par le patient P jusqu'au ventilateur 1 (sens de la flèche E).

Dans ce cas, les débranchements du circuit patient 2 peuvent se produire au niveau des connexions A1, A2 entre ventilateur 1 et les extrémités amont des branches 2a, 2b du circuit 2, et/ou des connexions D1, D2 entre les extrémités aval des branches 2a, 2b du circuit 2 et la pièce en Y 6, et/ou au niveau de la connexion B entre la pièce en Y 6 elle-même et l'interface respiratoire et/ou au niveau de la région de contact C entre interface respiratoire 3 et visage du patient P.

Il est indispensable de pouvoir détecter de tels débranchements du circuit patient 2 du ventilateur médical 1. Pour ce faire, le ventilateur médical 1 comprend des moyens d'alarme incluant une alarme de surveillance de l'état de débranchement du circuit 2. Lorsque celle-ci détecte une déconnexion du circuit 2, elle se lève et prévient le patient ou le personnel médical.

De façon générale, la détection d'un débranchement par le ventilateur 1 est un compromis entre la détection d'un plus grand nombre de bonnes détections, c'est-à-dire que le débranchement est détecté par le ventilateur dès déconnexion d'un élément du chemin d'air à l'une ou l'autre des différentes localisations possibles, comme expliqué ci-avant, et la minimisation des fausses détections, c'est-à-dire que le ventilateur détecte un débranchement alors que tous les éléments sont correctement raccordés. A l'inverse, il est aussi important de pouvoir détecter une reconnexion de tous les éléments constituant le chemin d'air entre le ventilateur 1 et le patient.

Selon la présente invention, pour répondre à ce problème de détection efficace de débranchement, le ventilateur médical est configuré pour opérer une estimation de la compliance, comme expliqué ci-après.

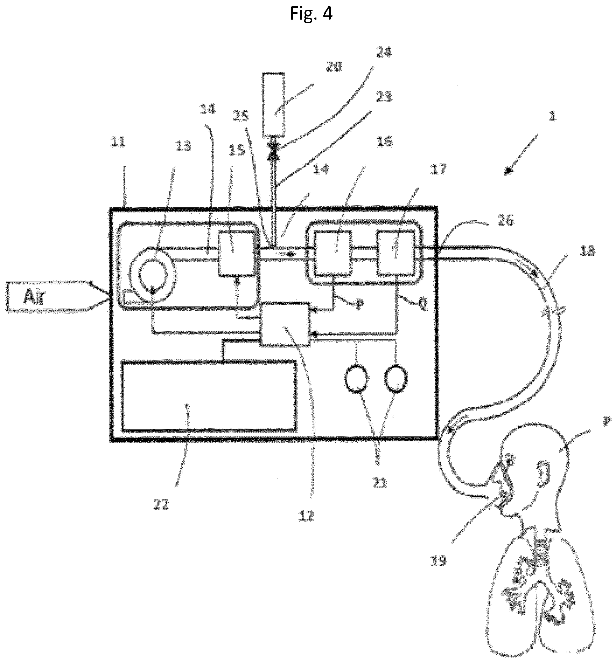

La Fig. 4 schématise un mode de réalisation d'un ventilateur médical 1 ou appareil d'assistance respiratoire permettant de réaliser une détection efficace de débranchement du circuit patient 18 basée sur la compliance.

Le ventilateur médical 1 de l'invention comprend une carcasse ou coque externe 12 dans laquelle est agencée une source de gaz 3, à savoir ici une micro-soufflante motorisée, c'est-à-dire équipée d'un moteur électrique animant une roue à ailettes, délivrant ici un flux d'air (teneur en oxygène 21% en vol.) dans un chemin de gaz, i.e. un circuit de gaz 14 interne, en communication fluidique avec la sortie d'air de la micro-soufflante 13.

Le circuit de gaz interne 14 comprend un ou des conduits ou passages de gaz, ou analogue, configuré pour convoyer le gaz au sein de la carcasse 11 du ventilateur 1 jusqu'à une sortie de gaz 26, aussi appelée sortie ventilateur.

Selon un autre mode de réalisation (non montré), la source de gaz 13 peut être une source externe du ventilateur 1, tel une alimentation en air comprimé, par exemple un conduit souple relié à une prise de distribution murale de gaz ou à un récipient de gaz sous pression, telle une bouteille de gaz sous pression.

L'air provenant de la source de gaz 13 est acheminé par le circuit de gaz interne 14 jusqu'à un patient P par l'intermédiaire d'un circuit patient 18, tel un conduit de gaz flexible, par exemple un (ou des) tuyaux souples en polymère, auquel il est administré au moyen d'une interface respiratoire 19, tel un masque nasal ou facial. Le circuit patient 18 vient se raccorder fluidiquement à la sortie de gaz 26 du ventilateur 1.

Le circuit patient 18 peut être à simple branche, comme illustré sur Fig. 4, ou à double branche (i.e. une branche inspiratoire et une branche expiratoire), comme celui illustré sur Fig. 3.

Un premier capteur de débit 15, un capteur de pression 16 et un second capteur de débit 17 sont agencés, en série, sur le circuit interne de gaz 14, en aval de la micro-soufflante 13, pour y opérer des mesures de pression P et de débit Q du gaz qui y circule. Le capteur de pression 16 est agencé entre le premier capteur de débit 15 et le second capteur de débit 17.

Des moyens de pilotage 12, c'est-à-dire une unité de traitement et de commande, comprenant typiquement une carte électronique comprenant un (ou des) microprocesseur, tel un microcontrôleur, mettant en œuvre au moins un algorithme, reçoit et traite les mesures (i.e. signaux) opérées par les capteurs de pression 16 et de débit 15, 17.

Les moyens de pilotage 12 commandent ici l'alimentation en gaz, c'est-à-dire ici la source de gaz 13 de type micro-soufflante motorisée, délivrant le flux d'air dans le circuit de gaz 14 de manière à délivrer un débit et/ou une pression de gaz selon les modalités du mode de ventilation sélectionné par le médecin, lesquels modes et modalités sont par exemple renseignées au moyen de boutons de réglages 21 et/ou d'un écran 22, de préférence tactile, formant une IHM ou interface homme-machine.

Les composants du ventilateur 1, notamment ici la micro-soufflante 13 motorisée, les capteurs de pression et de débit 15-17, au moins une partie du circuit de gaz 14 et les moyens de pilotage 12 sont agencés dans la carcasse externe 11 de l'appareil.

Par ailleurs, une seconde source de gaz 20 contenant de l'oxygène ou « gaz riche en oxygène », à savoir ici une bouteille d'oxygène ou une canalisation d'oxygène, est reliée fluidiquement au circuit de gaz 14 du ventilateur 1, via un ou plusieurs conduits de gaz 23, de manière à introduire dans le flux d'air circulant dans le circuit de gaz 14 du ventilateur 1, le gaz additionnel riche en oxygène, par exemple de l'oxygène pur (teneur en oxygène 100% en vol.).

Les moyens de pilotage 12 commandent la vanne 24 contrôlant l'arrivée du gaz riche en oxygène dans le circuit de gaz 14, par exemple une électrovanne pilotée. Dans un autre mode de réalisation, l'alimentation en gaz riche en oxygène n'est pas contrôlée par la vanne 24 mais maitrisée par l'utilisateur qui en détermine la présence ou l'absence et les caractéristiques telles que débit et/ou pression.

L'introduction du gaz riche en oxygène se fait en un site d'adjonction 25 situé entre le premier capteur de débit 15 et le capteur de pression 16. Alternativement, l'introduction du gaz riche en oxygène peut se faire en un site d'adjonction situé soit en amont de la micro-soufflante 13, soit au niveau de la sortie d'air de la micro-soufflante 13 (non représenté).

Comme déjà mentionné, le ventilateur 1 comprend également une interface homme-machine ou IHM comprenant par exemple touches, boutons rotatifs ou translatifs ou analogues 21, permettant à l'utilisateur d'entrer des informations ou des consignes dans le ventilateur 1, ou d'opérer des choix, des validations ou des sélections dans des menus par exemple. L'IHM comprend en outre un écran d'affichage 22, tel un écran digital tactile, permettant non seulement d'afficher différentes informations, données, pictogrammes, graphiques etc... mais aussi une saisie ou entrée de données en vue de leur utilisation, notamment par les moyens de pilotage 20, ou aussi d'opérer des choix, des sélections, des validations... de paramètres, modes de fonctionnement ou autres.

Bien entendu, le ventilateur 1 peut comprendre en outre des moyens d'alimentation en courant électrique (non montrés) comme un cordon et une prise de raccordement au secteur (110/220V), un transformateur de courant et/ou une batterie interne, alimentant les composants nécessitant du courant électrique pour fonctionner, notamment la micro-soufflante 13, notamment son moteur électrique, les moyens de pilotage 20, les capteurs 15-17, l'écran 22 de l'IHM ou tout autre composant.

Selon l'invention, les moyens de pilotage 12, en particulier leur microprocesseur, sont configurés pour estimer la compliance du patient P et du circuit patient 18, et en déduire un débranchement du circuit patient 18, à l'un ou l'autre des sites illustrés sur Fig. 1 à Fig. 3.

D'une façon générale, la compliance correspond à la capacité des poumons d'un individu à augmenter leur volume sous l'effet de la pression gazeuse qui leur est appliquée. Une compliance excessivement élevée suppose une cavité immense, donc au-delà de la capacité des poumons, signifie donc qu'un débranchement du circuit patient 18 s'est produit.

Autrement dit, selon l'invention, on détecte tout débranchement du circuit patient 18 en utilisant le critère de la compliance mesurée et en déduisant qu'un tel débranchement s'est produit lorsque que le microprocesseur détermine que la compliance devient supérieure à un seuil donné, notamment lorsqu'elle tend vers l'infini.

Pour ce faire, le processeur des moyens de pilotage 12 est configuré pour estimer, i.e. calculer, la compliance (C) du patient P et du circuit patient 18, notamment à partir de mesures opérées par les capteurs de débit 15, 16 et le capteur de pression 17.

L'équation de mouvement, appliquée au patient, mis en œuvre dans le processeur des moyens de pilotage 12 est la suivante :

$$Pprox(t) = Rp.Q(t) + V(t)/C + Pmusc(t)$$

où :

- Pprox est la pression du gaz à l'entrée des voies aériennes du patient. Elle est estimée à partir de la pression mesurée par le capteur 16 de la Fig. 2, du débit mesuré par le capteur 17 de la Fig. 2 et de la résistance du circuit patient,
- Rp est la résistance du système respiratoire du patient,
- Q(t) est le débit mesuré par le capteur de débit 17 de Fig. 2,
- V(t) est le volume courant délivré au patient à chaque instant t,
- C est la compliance du système respiratoire du patient, et
- Pmusc(t) est la pression musculaire générée par le patient pour inspirer.

En appliquant l'équation entre la fin de l'inspiration (FinI) et le début de l'inspiration (DebI), on a alors :

$$Pprox(FinI) - Pprox(DebI) = Rp.(Q(FinI) - Q(DebI))+((V(FinI)-V(DebI)) / C) +Pmusc(FinI)-Pmusc(DebI)$$

Au début et à la fin de chaque phase expiratoire du patient P, on considère que le débit de gaz est nul (approximation), i.e. Q(FinI) = Q($Deb_{Inspi}$) = 0, et que l'effort du patient est nul (approximation), i.e. Pmusc(FinI) = Pmusc(DebI) = 0.

De plus, la différence de volume entre le début et la fin d'une phase inspiratoire correspond à Vti, i.e. le volume délivré au patient pendant la phase inspiratoire.

On obtient alors :

$$Pprox(Fin_{Inspi}) - Pprox(Deb_{Inspi}) = Vti / C(t)$$

Donc :

$$C(t) = \frac{Vti}{P_{prox}(Fin_{inspi}) - P_{prox}(Deb_{inspi})}$$

Où :

- $P_{prox}(Fin_{inspi})$ est la pression proximale en fin d'inspiration,
- $P_{prox}(Deb_{inspi})$ est la pression proximale en début d'inspiration, et
- V*ti* est le volume de gaz délivré au patient sur 1 cycle ventilatoire, c'est-à-dire pendant la durée de la phase inspiratoire pendant laquelle du gaz est délivré au patient.

Typiquement, cette équation est mise en œuvre dans le processeur des moyens de pilotage 12 pour déterminer la compliance calculée C(t).

La pression patient Pprox est estimée à partir de la pression et du débit amont mesurés par les capteurs de débit 17 et de pression 16. En prenant en compte la perte de charge, c'est-à-dire la résistance Rc, générée par le circuit patient 2, on a :

$$Pa(t) - Pprox(t) = Rc . Qa(t)$$

Où :

- Pa est la pression amont mesurée par le capteur 16 de la Fig. 2,
- Qa est le débit amont mesuré par le capteur 17 de la Fig. 2,
- Pprox est la pression proximale, i.e. patient.

Par ailleurs, le volume de gaz *Vti* délivré au patient pendant un cycle insufflatoire du ventilateur est obtenu par intégration sur cette durée, de la ou des valeurs de débit Q(t) mesurées par le capteur de débit 17, par exemple sur une durée de cycle insufflatoire comprise entre environ 2 à 4 secondes.

Ensuite, le processeur compare la compliance C calculée en fin d'inspiration, appelée C(t), à une valeur-seuil donnée, à savoir une valeur maximale de compliance ($C_{max}$), afin de détecter un problème de débranchement du circuit patient 18, lorsque la compliance calculée C(t) excède ladite valeur maximale de compliance ($C_{max}$), c'est-à-dire lorsque le processeur détermine que : C(t) > $C_{max}$.

Stricto sensu, la compliance calculée C(t) correspond à une compliance discrète calculée en fin d'inspiration, c'est-à-dire correspondant à 1 cycle respiratoire.

La valeur-seuil maximale de compliance ($C_{max}$) peut dépendre du patient P traité puisque la capacité et le fonctionnement des poumons dépendent notamment de l'âge du patient. Il est donc avantageux de pouvoir fixer une valeur-seuil maximale de compliance ($C_{max}$) différente selon le patient P traité.

A cette fin, l'IHM est configurée pour permettre à l'utilisateur, tel un personnel soignant, tel un médecin ou analogue, de sélectionner une catégorie de patient, à savoir adulte, enfant ou nourrisson, et donc de pouvoir sélectionner ou fixer la valeur-seuil maximale de compliance ($C_{max}$) la plus adéquate pour le patient considéré.

Le choix peut se faire via les moyens de sélection 21, tels un ou des organes de sélection 21 manuelle, tels que boutons, touches ou analogues, et/ou l'écran 22, notamment via une (ou des) touche(s) tactiles affichées à l'écran 22.

La valeur-seuil maximale de compliance ($C_{max}$) peut être soit un paramètre entré par l'utilisateur, i.e. personnel soignant, soit être sélectionné parmi des choix affichés à l'écran, soit être une valeur fixée par défaut dès que la catégorie de patient a été choisie.

Typiquement, la valeur-seuil maximale de compliance ($C_{max}$) est comprise entre 20 et 600 mL/cmH2O selon le ventilateur, le circuit patient et le patient considérés.

Ainsi, par exemple, on peut utiliser les valeurs-seuils maximales de compliance ($C_{max}$) suivantes selon la catégorie de patient sélectionnée :

- Adulte : $C_{max}$ = 300 mL/cmH2O
- Enfant : $C_{max}$ = 200 mL/cmH2O
- Nourrisson : $C_{max}$ = 100 mL/cmH2O

Si la compliance calculée par le processeur dépasse ce seuil $C_{max}$, le processeur détermine qu'un débranchement a lieu et déclenche alors une alarme sonore et/ou visuelle, par exemple un message d'alarme s'affichant sur l'écran 22 de l'IHM, l'allumage d'un dispositif d'alerte lumineux, tels une ou des LEDs par exemple, et/ou un signal sonore.

Le ventilateur comprend aussi des moyens pour limiter les faux-positifs, notamment lorsque le patient fait de grosses inspirations, en prenant en compte la pente de pression pendant l'inspiration, voire aussi de la pente de débit.

En effet, en ventilation normale ou lors d'un débranchement du patient, la pente de pression est positive ou nulle. Lorsque le patient demande plus que le réglage du ventilateur, par exemple lorsqu'il fait un soupir ou un profond appel de gaz, de type « grosse » inspiration ou inspiration d'un volume gazeux anormalement élevé, la pente de pression devient négative (car elle décroit en fonction du temps) sans pour autant, signifier un débranchement du circuit. De manière analogue, la pente de débit peut devenir positive sans pour autant, signifier un débranchement du circuit.

Dans ce cas, malgré l'augmentation importante de la compliance mesurée, le processeur est configuré pour ne pas déclencher l'alarme de débranchement en compliance.

Par ailleurs, le processeur peut être aussi configuré pour détecter un rebranchement en compliance lorsqu'il détermine que C(t) < Cmax. Le processeur considère alors que le circuit est rebranché selon le critère de compliance. En réponse à cette détection du rebranchement, le processeur va stopper l'alarme que le processeur a déclenché après détection du débranchement.

D'une façon générale, un ventilateur médical selon l'invention permet de délivrer une aide respiratoire, c'est-à-dire une ventilation artificielle, à un patient souffrant de troubles ou insuffisances respiratoires plus ou moins sévères, pouvant résulter de différentes pathologies ou analogues, en particulier à un patient ventilé en milieu hospitalier, pendant plusieurs jours, même plusieurs semaines ou plusieurs mois.

**Revendications**

**1.** Ventilateur médical (1) comprenant :

- un circuit de gaz interne (14) pour acheminer du gaz comprenant une sortie de gaz (26),
- au moins un capteur de débit (15, 17) et au moins un capteur de pression (16) agencés sur le circuit de gaz interne (14) pour opérer des mesures de débit et de pression au sein dudit circuit de gaz interne (14),
- des moyens de pilotage (12) à au moins un microprocesseur configurés pour traiter les mesures de débit et de pression opérées par lesdits capteurs de débit (15, 17) et de pression (16),
- un circuit patient (18) raccordé fluidiquement à la sortie de gaz (26) du circuit de gaz interne (14), et
- une interface respiratoire (19) raccordée fluidiquement au circuit patient (18),

**caractérisé en ce que** les moyens de pilotage (12) sont configurés pour :

a) déterminer une valeur de compliance C(t) à partir d'au moins des mesures de débit et de pression opérées par lesdits capteurs de débit (15, 17) et de pression (16),
b) comparer la valeur de compliance C(t) déterminée à une valeur de compliance maximale ($C_{max}$) donnée,
c) déterminer un débranchement du circuit patient (18) ou de l'interface respiratoire (19) lorsque la valeur de compliance C(t) est supérieure ou égale à la valeur de compliance maximale ($C_{max}$) donnée, et
d) déclencher une alarme lorsqu'un débranchement du circuit patient (18) ou de l'interface respiratoire (19) est déterminé.

**2.** Ventilateur selon la revendication 1, **caractérisé en ce qu'**il comprend des moyens de fixation ou de sélection de compliance maximale pour fixer ou sélectionner une valeur de compliance maximale ($C_{max}$).

3. Ventilateur selon l'une des revendications 1 ou 2, **caractérisé en ce qu'**il comprend des moyens de sélection de catégorie de patient (21, 22) permettant de sélectionner une catégorie de patient choisie parmi adulte, enfant et nourrisson, ladite sélection de catégorie de patient conduisant à une sélection automatique d'une valeur de compliance maximale ($C_{max}$).

4. Ventilateur selon la revendication 1, **caractérisé en ce que** les moyens de pilotage (12) sont configurés pour calculer la valeur de compliance C(t) à partir de la formule suivante :

$$C(t) = \frac{Vti}{P_{prox}(Fin_{inspi}) - P_{prox}(Deb_{inspi})}$$

où :

- $P_{prox}(Fin_{inspi})$ est la pression proximale en fin d'inspiration,
- $P_{prox}(Deb_{inspi})$ est la pression proximale en début d'inspiration, et
- V*ti* est le volume de gaz délivré au patient sur 1 cycle ventilatoire.

5. Ventilateur selon la revendication 1, **caractérisé en ce que** les moyens de pilotage (12) comprennent au moins un microprocesseur agencé sur une carte électronique.

6. Ventilateur selon la revendication 3, **caractérisé en ce que** les moyens de sélection de catégorie de patient (21, 22) comprennent un organe de sélection manuelle (21) et/ou un écran d'affichage (22).

7. Ventilateur selon la revendication 1, **caractérisé en ce que** le circuit patient (18) comprend un ou deux conduits de gaz agencés en parallèle (i.e. circuit à une ou deux branches).

8. Ventilateur selon la revendication 1, **caractérisé en ce que** le circuit patient (18) est relié directement à interface respiratoire (19) ou indirectement via une pièce de raccordement, typiquement une pièce en Y.

9. Ventilateur selon la revendication 1, **caractérisé en ce que** la source de gaz (13) est une micro-soufflante motorisée.

10. Ventilateur selon la revendication 2, **caractérisé en ce que** les moyens de fixation ou de sélection de compliance maximale sont configurés pour fixer ou sélectionner une valeur de compliance maximale ($C_{max}$) comprise entre 20 et 600 mL/cmH2O.

11. Ventilateur selon l'une des revendications 1, 2 ou 10, **caractérisé en ce qu'**il comprend en outre des moyens de mémorisation configurés pour mémoriser au moins une valeur de compliance maximale ($C_{max}$).

12. Ventilateur selon l'une des revendications 1 ou 4, **caractérisé en ce que** les moyens de pilotage (12) sont configurés pour calculer un volume de gaz *(Vti)* délivré au patient pendant la durée d'un cycle ventilatoire à partir des mesures de débit (Q) opérées durant ledit cycle ventilatoire.

13. Ventilateur selon la revendication 12, **caractérisé en ce que** les moyens de pilotage (12) sont configurés pour déterminer le volume de gaz *(Vti)* par intégration des mesures de débit (Q) durant ledit cycle ventilatoire.

14. Ventilateur selon la revendication 1, **caractérisé en ce que** les moyens de pilotage (12) sont configurés pour déclencher une alarme sonore et/ou visuelle en cas de débranchement du circuit.

15. Ventilateur selon l'une des revendications 4, 12 ou 13, **caractérisé en ce que** la durée d'un cycle ventilatoire est comprise entre 1 et 5 secondes environ.

**Patentansprüche**

1. Medizinisches Beatmungsgerät (1), umfassend:

- einen internen Gaskreis (14) zum Befördern von Gas, der einen Gasauslass (26) umfasst,
- mindestens einen Durchflussmengensensor (15, 17) und mindestens einen Drucksensor (16), die an dem internen Gaskreis (14) angeordnet sind, um Durchflussmengen- und Druckmessungen in dem internen Gaskreis (14) vorzunehmen,
- Ansteuerungsmittel (12) mit mindestens einem Mikroprozessor, die dazu ausgelegt sind, die von den Durchflussmengensensoren (15, 17) und Drucksensoren (16) vorgenommenen Durchflussmengen- und Druckmessungen zu verarbeiten,
- einen Patientenkreis (18), der fluidisch an den Gasauslass (26) des internen Gaskreises (14) angeschlossen ist, und
- eine Atemschnittstelle (19), die fluidisch an den Patientenkreis (18) angeschlossen ist,

**dadurch gekennzeichnet, dass** die Ansteuerungsmittel (12) dazu ausgelegt sind:

a) einen Compliance-Wert C(t) ausgehend mindestens von den von den Durchflussmengensensoren (15, 17) und Drucksensoren (16) vorgenommenen Durchflussmengen- und Druckmessungen zu bestimmen,
b) den bestimmten Compliance-Wert C(t) mit einem gegebenen maximalen Compliance-Wert ($C_{max}$) zu vergleichen,
c) eine Trennung des Patientenkreises (18) oder der Atemschnittstelle (19) zu bestimmen, wenn der Compliance-Wert C(t) größer als oder gleich dem gegebenen maximalen Compliance-Wert ($C_{max}$) ist, und
d) einen Alarm auszulösen, wenn eine Trennung des Patientenkreises (18) oder der Atemschnittstelle (19) bestimmt wird.

2. Beatmungsgerät nach Anspruch 1, **dadurch gekennzeichnet, dass** es Mittel zum Festlegen oder Auswählen einer maximalen Compliance umfasst, um einen maximalen Compliance-Wert ($C_{max}$) festzulegen oder auszuwählen.

3. Beatmungsgerät nach einem der Ansprüche 1 oder 2, **dadurch gekennzeichnet, dass** es Mittel zum Auswählen einer Patientenkategorie (21, 22) umfasst, die es ermöglichen, eine unter Erwachsener, Kind und Säugling gewählte Patientenkategorie auszuwählen, wobei das Auswählen der Patientenkategorie zu einem automatischen Auswählen eines maximalen Compliance-Werts ($C_{max}$) führt.

4. Beatmungsgerät nach Anspruch 1, **dadurch gekennzeichnet, dass** die Ansteuerungsmittel (12) dazu ausgelegt sind, den Compliance-Wert C(t) ausgehend von der folgenden Formel zu berechnen:

$$C(t) = \frac{Vti}{P_{prox}(Ende_{Einatm}) - P_{prox}(Anf_{Einatm})}$$

worin:

■ $P_{prox}(Ende_{Einatm})$ der proximale Druck am Ende einer Einatmung ist,
■ $P_{prox}(Anf_{Einatm})$ der proximale Druck am Anfang einer Einatmung ist,
■ $Vti$ das über 1 Beatmungszyklus an den Patienten abgegebene Gasvolumen ist.

5. Beatmungsgerät nach Anspruch 1, **dadurch gekennzeichnet, dass** die Ansteuerungsmittel (12) mindestens einen Mikroprozessor umfassen, der auf einer Elektronikkarte angeordnet ist.

6. Beatmungsgerät nach Anspruch 3, **dadurch gekennzeichnet, dass** die Mittel zum Auswählen einer Patientenkategorie (21, 22) ein Element zum manuellen Auswählen (21) und/oder einen Anzeigebildschirm (22) umfassen.

7. Beatmungsgerät nach Anspruch 1, **dadurch gekennzeichnet, dass** der Patientenkreis (18) eine oder zwei parallel angeordnete Gasleitungen (d. h. einen Kreis mit einem oder zwei Zweigen) umfasst.

8. Beatmungsgerät nach Anspruch 1, **dadurch gekennzeichnet, dass** der Patientenkreis (18) mit der Atemschnittstelle (19) direkt verbunden ist oder indirekt über ein Anschlussstück, typischerweise ein Y-Stück.

9. Beatmungsgerät nach Anspruch 1, **dadurch gekennzeichnet, dass** die Gasquelle (13) ein motorisiertes Mikrogebläse ist.

10. Beatmungsgerät nach Anspruch 2, **dadurch gekennzeichnet, dass** die Mittel zum Festlegen oder Auswählen einer maximalen Compliance dazu ausgelegt sind, einen maximalen Compliance-Wert ($C_{max}$) zwischen 20 und 600 mL/cmH2O festzulegen oder auszuwählen.

11. Beatmungsgerät nach einem der Ansprüche 1, 2 oder 10, **dadurch gekennzeichnet, dass** es ferner Speichermittel umfasst, die dazu ausgelegt sind, mindestens einen maximalen Compliance-Wert ($C_{max}$) zu speichern.

12. Beatmungsgerät nach einem der Ansprüche 1 oder 4, **dadurch gekennzeichnet, dass** die Ansteuerungsmittel (12) dazu ausgelegt sind, ein während der Dauer eines Beatmungszyklus an den Patienten abgegebenes Gasvolumen (V*ti*) ausgehend von den während des Beatmungszyklus vorgenommenen Durchflussmengenmessungen (Q) zu berechnen.

13. Beatmungsgerät nach Anspruch 12, **dadurch gekennzeichnet, dass** die Ansteuerungsmittel (12) dazu ausgelegt sind, das Gasvolumen (V*ti*) durch Integration der Durchflussmengenmessungen (Q) während des Beatmungszyklus zu bestimmen.

14. Beatmungsgerät nach Anspruch 1, **dadurch gekennzeichnet, dass** die Ansteuerungsmittel (12) dazu ausgelegt sind, im Fall einer Trennung des Kreises einen akustischen und/oder visuellen Alarm auszulösen.

15. Beatmungsgerät nach einem der Ansprüche 4, 12 oder 13, **dadurch gekennzeichnet, dass** die Dauer eines Beatmungszyklus ungefähr zwischen 1 und 5 Sekunden beträgt.

**Claims**

1. Medical ventilator (1) comprising:

    - an internal gas circuit (14) for conveying gas, comprising a gas outlet (26),
    - at least one flowrate sensor (15, 17) and at least one pressure sensor (16), which are arranged on the internal gas circuit (14) in order to perform flowrate and pressure measurements within said internal gas circuit (14),
    - control means (12) with at least one microprocessor, which are configured to process the flowrate and pressure measurements performed by said flowrate (15, 17) and pressure (16) sensors,
    - a patient circuit (18) fluidically connected to the gas outlet (26) of the internal gas circuit (14), and
    - a respiratory interface (19) fluidically connected to the patient circuit (18),

    **characterized in that** the control means (12) are configured to:

    a) determine a compliance value C(t) from at least flowrate and pressure measurements performed by said flowrate (15, 17) and pressure (16) sensors,
    b) compare the determined compliance value C(t) to a given maximum compliance value ($C_{max}$),
    c) determine a disconnection of the patient circuit (18) or of the respiratory interface (19) when the compliance value C(t) is greater than or equal to the given maximum compliance value ($C_{max}$), and
    d) trigger an alarm when a disconnection of the patient circuit (18) or of the respiratory interface (19) is determined.

2. Ventilator according to Claim 1, **characterized in that** it comprises maximum compliance setting or selection means for setting or selecting a maximum compliance value ($C_{max}$).

3. Ventilator according to either of Claims 1 and 2, **characterized in that** it comprises patient category selection means (21, 22) for selecting a patient category chosen from adult, child and infant, said patient category selection leading to an automatic selection of a maximum compliance value ($C_{max}$).

4. Ventilator according to Claim 1, **characterized in that** the control means (12) are configured to calculate the compliance value C(t) from the following formula:

$$C(t) = \frac{Vti}{P_{prox}(End_{inspi}) - P_{prox}(Start_{inspi})}$$

where:

- $P_{prox}(End_{inspi})$ is the proximal pressure at the end of inspiration,
- $P_{prox}(Start_{inspi})$ is the proximal pressure at the start of inspiration, and
- $Vti$ is the volume of gas delivered to the patient over 1 ventilatory cycle.

5. Ventilator according to Claim 1, **characterized in that** the control means (12) comprise at least one microprocessor arranged on an electronic card.

6. Ventilator according to Claim 3, **characterized in that** the patient category selection means (21, 22) comprise a manual selection member (21) and/or a display screen (22).

7. Ventilator according to Claim 1, **characterized in that** the patient circuit (18) comprises one or two gas ducts arranged in parallel (i.e. circuit with one branch or two branches).

8. Ventilator according to Claim 1, **characterized in that** the patient circuit (18) is connected directly to the respiratory interface (19) or indirectly via a connecting piece, typically a Y-piece.

9. Ventilator according to Claim 1, **characterized in that** the gas source (13) is a motorized micro blower.

10. Ventilator according to Claim 2, **characterized in that** the maximum compliance setting or selection means are configured to set or select a maximum compliance value ($C_{max}$) of between 20 and 600 mL/cmH2O.

11. Ventilator according to any one of Claims 1, 2 and 10, **characterized in that** it further comprises storage means configured to store at least one maximum compliance value ($C_{max}$).

12. Ventilator according either of Claims 1 and 4, **characterized in that** the control means (12) are configured to calculate a gas volume ($Vti$) delivered to the patient for the duration of a ventilatory cycle from the flowrate measurements (Q) performed during said ventilatory cycle.

13. Ventilator according to Claim 12, **characterized in that** the control means (12) are configured to determine the gas volume ($Vti$) by integrating the flowrate measurements (Q) during said ventilatory cycle.

14. Ventilator according to Claim 1, **characterized in that** the control means (12) are configured to trigger an acoustic and/or visual alarm in the event of disconnection of the circuit.

15. Ventilator according to any one of Claims 4, 12 and 13, **characterized in that** the duration of a ventilatory cycle is between approximately 1 and 5 seconds.

Fig. 1

Fig. 2

Fig. 3

Fig. 4

## RÉFÉRENCES CITÉES DANS LA DESCRIPTION

**Documents brevets cités dans la description**

*   WO 8201654 A